# EUROPEAN PATENT APPLICATION

(11) **EP 1 428 889 A1**
(43) Date of publication of application: **16.06.2004**
(21) Application number: 02090399.3
(22) Date of filing: 10.12.2002
(51) Int. Cl.: C12Q 1/68

(54) **Method for monitoring the transition of a cell from one state into another**

(71) Applicant: Epigenomics AG, 10435 Berlin (DE)
(72) Inventor: The designation of the inventor has not yet been filed
(74) Representative: Schubert, Klemens, Dr.

(57) **Abstract**

The method relates to the field of molecular biology and cell biology. More specifically it is concerned with monitoring a cell's transition from one state into another with the use of a genome based technology. The method is based on a sufficient analysis of methylation patterns according to said cell states. In addition it includes the actual transition of said cell itself. This is done by exposing a cell to conditions expected to convert it from one state to another.

## Description

The method relates to the field of molecular biology and cell biology. More specifically it is concerned with monitoring a cell's transition from one state into another with the use of a genome based technology. The method is based on sufficient analysis of methylation patterns according to said cell states. In addition it includes the actual transition of said cell itself. This is done by exposing a cell to conditions expected to convert it from one state into another.

Tissue loss and end-stage organ failure pose major global health problems. Attempts to overcome these problems mainly rely on transplantation of tissues or whole organs. Those approaches are limited by the availability of donor organs and their possible immune rejection, situations unlikely to ameliorate.

Tissue-engineering allows treatment of tissue loss or organ failure by implantation of an engineered biological substitute, alone or in combination with synthetic devices.

Compared to the currently used transplantation technologies, one advantage of using tissue engineering products is the possibility to use autologous sources to develop the tissue from, and thus allowing to treat a patient with his own cells and hereby avoiding immune rejection. To provide these individualized products, the starting material to develop the ideal tissue from, needs to be obtained from the patient himself. This material needs to be quality assessed, cells need to be expanded and correctly differentiated. If the raw material is isolated from autologous sources, the process needs to be adapted to different starting material for each individual patient. In addition, it has to be guaranteed that the tissue product, a patient will be transplanted with, origins from the same patient's starting material.

In those areas where tissue engineering is already established, however, the standard tissue engineered product is from allogenic rather than from autologous sources. Nevertheless, individual end products need to be analyzed efficiently as it is of utmost importance for the patient that he receives a correctly engineered tissue, i.e. free of undifferentiated or incorrectly differentiated cells, which could cause the development of a tumor, or result in other unwanted effects. The exact lineage, functionality and homogeneity of the product must be guaranteed.

One potential starting material for tissue engineering products are stem cells and/ or other progenitor cells. More detailed definitions are given in the description of the invention. Generally, stem cells are cells that have the ability to reproduce themselves for indefinite periods - often throughout the life of the organism. Under the right conditions, or given the right signals, stem cells can give rise (differentiate) to the different cell types that make up the organism. Many of the terms used to define stem cells depend on their behavior in the intact organism (in vivo), under specific laboratory conditions (in vitro) or after transplantation.

The embryonic stem cell is defined by its origin, i.e. from the earliest stages of the development of the embryo, called the blastocyst. Specifically, embryonic stem cells are derived from the inner cell mass of the blastocyst at a stage before its implantation in the uterine wall.

An adult stem cell is an undifferentiated (unspecialized) cell that occurs in differentiated (specialized) tissues, renews itself, and becomes specialized to yield the different cell types of that specific tissue. In the adult, organ formation and regeneration was thought to occur through the action of organ- or tissue-restricted stem cells only (i.e. haematopoietic stem cells giving rise to all the cells of the blood, neural stem cells making neurons, astrocytes, and oligodendrocytes). However, it has been shown that stem cells from one organ, for example the haematopoietic stem cells can develop into the differentiated cells of another organ, such as the liver, brain or kidney. Adult stem cells are capable of making identical copies of themselves for the lifetime of the organism. In repair or regeneration events adult stem cells divide to generate progenitor or precursor cells, which then differentiate or develop into "mature" cell types that have characteristic shapes and specialized functions.

A progenitor or precursor cell occurs in fetal or adult tissues and is partially specialized; it divides and gives rise to differentiated cells. When a progenitor or precursor cell divides, it can form more progenitor or precursor cells or it can form two specialized cells. Usually these latter ones are not capable of replicating themselves. However, there are exceptions, for example specific octaploid cells in the liver, which can replicate themselves.

The term pluripotent refers to the capacity of a single cell to give rise to several different lineages of cells and renew itself. Pluripotency can occur at several levels. The penultimate pluripotent cell also called totipotent cell, is the fertilized egg, which can give rise to every cell in the body.

The capacity of stem cells for virtually unlimited self-renewal and differentiation capacity has opened up the prospect of widespread applications in biomedical research and regenerative medicine. For the latter, the cells provide hope that it will be possible to overcome problems of donor tissue shortage and also, by making the cells immunocompatible with the recipient, implant rejection. Human pluripotent cells are derived from pre-implantation embryos and differentiation can be encouraged towards particular cell lineages.

To illustrate the potential of tissue engineering with regard to cell lines developed from an adult stem cell, an example is given here that describes the approach to develop progenitor cells isolated from the pancreas into highly differentiated cells that secrete insulin in a glucose responsive manner with the aim to cure diabetes patients:

The pancreas contains two classes of cell types, exocrine and endocrine cells. During development the endocrine cells emerge from the pancreatic ducts and form aggregates that eventually form what is known as island of Langerhans. In humans, there are four types of islet cells: the insulin-producing beta-cell is one of them. Over the past several years doctors have attempted to cure diabetes by injecting patients with pancreatic islet cells. However, the islet cells must be immunologically compatible and the tissue must be freshly obtained from cadavers - within 8 h of death. Also usually several donors are required for a single surgery. These requirements are difficult to meet. Further, islet cell transplant recipients face a lifetime of immunosuppressant therapy.

To overcome these problems a different source of glucose responding insulin-producing beta cells has been identified in the field of tissue engineering: Suitable progenitor cells or stem cells can be induced to differentiate towards fully functioning beta cells. A number of approaches to control this specific cell differentiation are known. In one of them the starting material is adult tissue. Susan Bonner-Weir and her colleagues reported that cultured ductal cells, isolated from human pancreatic tissue, could be induced to differentiate into clusters that contained both ductal and endocrine cells, from which the latter produced insulin when exposed to glucose (Bonner-Weir S, Taneja M, Weir GC, Tatarkiewicz K, Song KH, Sharma A, O'Neil JJ. (2000) In vitro cultivation of human islets from expanded ductal tissue. Proc Natl Acad Sci USA 97, 7999-8004).

Another potential starting material for tissue engineering products are fully differentiated cells, which can be dedifferentiated in culture.

It can also be advantageous to re-differentiate a specific cell-type - from a fully differentiated cell to a less specialized cell, which potentially gives rise to another cell-type. For example, human chondrocytes, that have been cultured, de-differentiated and expanded are able to re-differentiate under controlled conditions. This ability has been shown to be enhanced by specific combinations of growth factors and hormones during 3D culture (Yaeger et al. (1997) Synergistic action of transforming growth factor-beta and insulin-like growth factor-I induces expression of type II collagen and aggrecan genes in adult human articular chondrocytes. Exp Cell Res. 237, 318-355; Jakob et al. (2001) Specific growth factors during the expansion and redifferentiation of adult human articular chondrocytes enhance chondrogenesis and cartilaginous tissue formation in vitro. J Cell Biochem 81, 368-77). Among those growth factors are TGF-beta, FGF and EGF, which have pronounced effects on the differentiation when supplemented to the culture media along with the required application of a scaffold to induce the re-differentiation.

A standard method to distinguish the differentiation stages of chondrocytes, is to determine the ratio of expression levels of the marker proteins collagen 2 and collagen 1 and/or the ratio of expression levels of aggrecan and versican. Both ratios are indicators for differentiation stages of chondrocytes.

However, a better method to distinguish specific tissues is the analysis of methylation states. It has been described to differentiate tissues such as prostate and kidney by Adorjan et al. (Tumour class prediction and discovery by microarray-based DNA methylation analysis. (2002) Nucleic Acids Res. 30, e21). The present invention is about how to use different methylation detection technologies to discriminate between differentiation stages of a cell or cells.

It is the aim of a number of companies to develop and engineer new cell and tissue types in vitro, from stem or progenitor cells in a reliable and reproducible manner, which will eventually gain regulatory approval. For this purpose it is required that
a) cells can be maintained and expanded without changing their phenotype and their differentiation status,
b) cells can be manipulated and differentiated in a targeted, standardized and efficient way in order to obtain the desired cell type and
c) exact lineage, functionality, homogeneity and differentiation status can be assessed.

In early steps of differentiation and growth experiments result assessment addresses questions as to whether correct progenitor cells were chosen or whether differentiation pathways are the anticipated ones and are likely to yield correct tissues. In more advanced stages of product development, the assessment is required for the proof of product quality. In this context "correct" is understood as fully biologically functioning regarding the cell type in question.

So far, the state of the art in assessing those requirements, as described above, is based on the analysis of phenotypic changes, such as morphological and biochemical changes of said cells. Typical technologies in use are immuno-histochemical analyses, fluorescent activated cell sorting (FACS) and expression analysis of specific marker proteins. Those biochemical assays often are inconclusive, lengthy, time consuming and not fit for high throughput analyses. They do not always qualify for a prediction of the intended cellular function and are often only meaningful at the end of the differentiation process.

A standard method to determine a cell's state is the use of immuno-histochemical assays. These are based on the detection of specified proteins. A recent publication describes the use of several markers suitable for the differentiation of osteoarthritic chondrocytes (Pfander D, Swoboda and Kirsch T (2001) Expression of early and late differentiation markers (proliferating cell nuclear antigen, syndecan-3, annexin VI and alkaline phosphatase. Am. J. Pathol 159: 1777-1783). Three proteins appear to be specific for early differentiation states and two different ones for late differentiation. However, their use as markers is restricted to the rather labor-intensive procedure of immunostaining. Generally, one assay per protein is required to monitor the expression of those proteins. Interpretation of these data is often complicated and results usually show only relative changes of expression. Making a defined statement about the cellular status becomes difficult, as it might be determined by a rather complex protein expression pattern.

The more marker proteins are known the more precisely a cell's differentiation status can be determined. Without the use of molecular biology techniques, such as RNA based cDNA/oligo-microarrays or a complex proteomics experiment, which enable the simultaneous view on a higher number of changes, cell differentiation itself and effects of growth factors on differentiation can hardly be studied in detail.

While proteomic approaches have not mastered basic difficulties, such as reaching sufficient sensitivity, approaches using RNA-based techniques to analyze expression patterns are well-known and widely used. Microarray-based expression analysis studies on cell differentiation is a growing area of research. It is recognized that precise patterns of differentially expressed genes ultimately direct a particular cell toward a given lineage and many of these are regulated during the earliest stages of differentiation.

A growing number of publications describes the use of microarray analysis for studying the differentiation of cells. For example:
Burton GR, Guan Y, Nagarajan R, McGehee RE (2002) Microarray analysis of gene expression during early adipocyte differentiation. Gene 293: 21-31.
Lu SJ, Quan C, Li F, Vida L and Honig GR (2002) Hematopoietic Progenitor Cells Derived from Embryonic Stem Cells: Analysis of Gene Expression. Stem Cell 20 (5): in press
Another group of scientists (Bruce Lahn et al.) is trying to understand the molecular mechanisms controlling cell differentiation and migration during the early stages of brain formation. They also try to identify the defining molecular features that distinguish stem cells from differentiated cells
(http://www.genes.uchicago.edu/fri/lahnres.html as by August 2002).

Both cDNA arrays and (oligonucleotide-based-) affymetrix chips allow a complex and sensitive analysis of changes in the expression pattern of cells. However, the decisive drawback of these technologies is their dependency on RNA. Despite extensive research with RNA, the general problem of its instability is not solved. Therefore, each single experiment with RNA needs to take into account that degradation of RNA will occur along the experimental procedure. This problem is aggravated by the fact that RNA expression levels change gradually, so that - for the majority of genes - the actual expression changes are overlapped and blurred by changes through random degradation.

Regulatory agencies are currently not willing to accept a technology platform relying on an expression microarray due to the shortcomings named above. In contrast, the method being subject of this invention is based on the stable DNA molecule rather than on easily degradable RNA molecules, and depends on a digital 0/1 signal (caused by a base being either methylated or not). Therefore, results are more sensitive and reliable than for RNA-dependent technologies. A platform based on this technology is also likely to be accepted by regulatory authorities.

In recent decades in molecular biology studies have focused primarily on genes, the transcription of those genes into RNA, and the translation of the RNA into protein. There has been a more limited analysis of the regulatory mechanisms associated with gene control. Gene regulation, for example, at what stage of development of the individual a gene is activated or inhibited, and the tissue specific nature of this regulation is less understood. However, it can be correlated with a high degree of probability to the extent and nature of methylation of the gene or genome. Specific cell types can be correlated with specific methylation patterns and this has been shown for a number of cases (Adorjan et al. (2002) Tumour class prediction and discovery by microarray-based DNA methylation analysis. Nucleic Acids Res. 30 (5) e21). Furthermore, this invention discloses a method on how to determine the state of a cell in a differentiation process by analyzing its methylation pattern.

In higher order eukaryotes DNA is methylated nearly exclusively at cytosines located 5' to guanine in the CpG dinucleotide. This modification has important regulatory effects on gene expression, especially when involving CpG rich areas, known as CpG islands, located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females.

The cytosine's modification in form of methylation contains significant information. It is obvious that the identification of 5-methylcytosine in a DNA sequence as opposed to unmethylated cytosine is of greatest importance to analyze its role further. But, because the 5-methylcytosine behaves just as a cytosine for what concerns its hybridization preference (a property relied on for sequence analysis) its positions can not be identified by a normal sequencing reaction.

Furthermore, in any amplification, such as a PCR amplification, this relevant epigenetic information, methylated cytosine or unmethylated cytosine, will be lost completely.

Several methods are known that solve this problem. Usually genomic DNA is treated with a chemical or enzyme leading to a conversion of the cytosine bases, which consequently allows to differentiate the bases afterwards. The most common methods are a) the use of methylation sensitive restriction enzymes capable of differentiating between methylated and unmethylated DNA and b) the treatment with bisulfite. The use of said enzymes is limited due to the selectivity of the restriction enzyme towards a specific recognition sequence.
Therefore, the specific reaction of bisulfite with cytosine, which, upon subsequent alkaline hydrolysis, is converted to uracil, whereas 5-methylcytosine remains unmodified under these conditions (Shapiro et al. (1970) Nature 227: 1047 ) is currently the most frequently used method for analyzing DNA for 5-methylcytosine. Uracil corresponds to thymine in its base pairing behavior, that is it hybridizes to adenine; whereas 5-methylcytosine does not change its chemical properties under this treatment and therefore still has the base pairing behavior of a cytosine, that is hybridizing with guanine. Consequently, the original DNA is converted in such a manner that 5-methylcytosine, which originally could not be distinguished from cytosine by its hybridization behavior, can now be detected as the only remaining cytosine using "normal" molecular biological techniques, for example, amplification and hybridization or sequencing. All of these techniques are based on base pairing, which can now be fully exploited. Comparing the sequences of the DNA with and without bisulfite treatment allows an easy identification of those cytosines that have been unmethylated.

An overview of the further known methods of detecting 5-methylcytosine may be gathered from the following review article: Rein T, DePamphilis ML, Zorbas H (1998), Nucleic Acids Res., 26: 2255.

In terms of sensitivity, the prior art is defined by a method, which encloses the DNA to be analyzed in an agarose matrix, thus preventing diffusion and renaturation of the DNA (bisulfite reacts with single-stranded DNA only), and which replaces all precipitation and purification steps with fast dialysis (Olek A, Oswald J, Walter J. (1996) A modified and improved method for bisulfite based cytosine methylation analysis. Nucleic Acids Res. 24: 5064-6). Using this method, it is possible to analyze individual cells, which illustrates the potential of the method.

To date, barring few exceptions (e.g., Zeschnigk M, Lich C, Buiting K, Doerfler W, Horsthemke B. (1997) A single-tube PCR test for the diagnosis of Angelman and Prader-Willi syndrome based on allelic methylation differences at the SNRPN locus. Eur J Hum Genet. 5: 94-8) the bisulfite technique is only used in research. Always, however, short, specific fragments of a known gene are amplified subsequent to a bisulfite treatment and either completely sequenced (Olek A, Walter J. (1997) The pre-implantation ontogeny of the H19 methylation imprint. Nat Genet. 3: 275-6) or individual cytosine positions are detected by a primer extension reaction (Gonzalgo ML and Jones PA. (1997) Rapid quantitation of methylation differences at specific sites using methylation-sensitive single nucleotide primer extension (Ms-SNuPE). Nucleic Acids Res. 25 :2529-31, WO 95/00669) or by enzymatic digestion (Xiong Z, Laird PW. (1997) COBRA: a sensitive and quantitative DNA methylation assay. Nucleic Acids Res. 25: 2535-4).

Another technique to detect hypermethylation is the so-called methylation specific PCR (MSP) (Herman JG, Graff JR, Myohanen S, Nelkin BD and Baylin SB. (1996), Methylation-specific PCR: a novel PCR assay for methylation status of CpG islands. Proc Natl Acad Sci U S A. 93: 9821-6). The technique is based on the use of primers that differentiate between a methylated and a non-methylated sequence if applied after bisulfite treatment of said DNA sequence. The primer either contains a guanine at the position corresponding to the cytosine in which case it will after bisulfite treatment only bind if the position was methylated. Or the primer contains an adenine at the corresponding cytosine position and therefore only binds to said DNA sequence after bisulfite treatment if the cytosine was unmethylated and has hence been altered by the bisulfite treatment so that it hybridizes to adenine. With the use of these primers, amplicons can be produced specifically depending on the methylation status of a certain cytosine and will as such indicate its methylation state.

Another new technique is the detection of methylation via Taqman PCR, also known as MethylLight (WO 00/70090). With this technique it became feasible to determine the methylation state of single or of several positions directly during PCR, without having to analyze the PCR products in an additional step.

In addition, detection by hybridization has also been described (Olek et al., WO 99/28498).

Further publications dealing with the use of the bisulfite technique for methylation detection in individual genes are: Grigg G, Clark S. (1994) Sequencing 5-methylcytosine residues in genomic DNA. Bioessays 16: 431-6; Zeschnigk M, Schmitz B, Dittrich B, Buiting K, Horsthemke B, Doerfler W. (1997) Imprinted segments in the human genome: different DNA methylation patterns in the Prader-Willi/Angelman syndrome region as determined by the genomic sequencing method. Hum Mol Genet. 6, 387-395; Feil R, Charlton J, Bird AP, Walter J, Reik W (1994) Methylation analysis on individual chromosomes: improved protocol for bisulphite genomic sequencing. Nucleic Acids Res. 22, 695-696; Martin V, Ribieras S, Song-Wang X, Rio MC, Dante R (1995) Genomic sequencing indicates a correlation between DNA hypomethylation in the 5' region of the pS2 gene and its expression in human breast cancer cell lines. Gene 157, 261-264; WO 97/46705, WO 95/15373 and WO 97/45560.

All of the documents cited herein are hereby incorporated by reference.

### Problem and Solution

To be able to maintain and expand cells without changing their phenotype and their original differentiation it is necessary to analyze those changes as fast, efficient and thorough as possible. The earlier changes can be detected the easier it is to refer back to the change's cause, which could be eliminated. Therefore a tool is required to monitor the occurrence of such changes resulting from unsuitable conditions to maintain said cells or simply from aging of said cell cultures. An early detection saves time and effort because cells can be discarded earlier.

To manipulate and differentiate cells in a targeted and efficient way a method is required that provides fast access to information about the cells' differentiation state. To be able to standardize this process, the required method needs to deliver reproducible data and needs to be easy to perform.

Such a method is provided by this invention. The method provides the means to transfer a cell from one state to another under controlled conditions and to monitor said transition. That way the effect of these conditions on the cells differentiation status can be analyzed. The method also provides the means for a detailed characterization of cells along their differentiation pathways. It is described how the exact lineage, functionality, homogeneity and differentiation status of a cell can be assessed with the means of methylation analysis.

Different cell types and differentiation statuses have distinct and specific methylation patterns. These methylation patterns are determined and used to select targeted cells or correctly differentiated cell types. When cells do not follow a specified differentiation pathway their cellular methylation pattern differs from those cells that do. This is easily detected and cell cultures could be erased respectively.

The method that is subject of this invention offers a tool how to monitor these cells during their attempted differentiation process, to quality assess if a cell is fully differentiated and hence fully functioning.

### Description of the invention

The method relates to the field of molecular biology and cell biology. More specifically it is concerned with monitoring a cell's transition from one state into another with the use of a genome based technology. The method is based on the analysis of methylation patterns according to said cell states. In addition, it includes the actual transition of said cell itself. This is done by exposing a cell to conditions expected to convert it from one state into another.

The present invention employs the following definitions.

"Differentiation" is the process by which a cell becomes more specialized, it loses some of its broader potency and gains cell-type specific characteristics: During differentiation an unspecialized cell, such as a stem cell, an early embryonic cell or a progenitor cell acquires the features of a specialized cell such as a heart, liver or muscle cell. In other words, it becomes specialized into one of the many cells that make up the body. During differentiation, certain genes become activated and other genes become inactivated in an intricately regulated fashion. As a result, a differentiated cell develops specific structures and performs certain functions.

The full process of differentiation occurring at development starts at a potent stem cell, leads via an intermediate cell, with limited differentiation potential, towards a highly specialized cell, not capable of or only slowly replicating itself.

A "stem cell" is a cell from the embryo, fetus or adult that has, under certain conditions, the ability to reproduce itself for long periods or, in the case of adult stem cells, throughout the life of the organism. Embryonic stem cells, in the laboratory, can proliferate indefinitely, a property not shared by adult stem cells. When a stem cell divides, one of the two new cells is often a stem cell capable of replicating itself again. However, sometimes both of the new cells are stem cells capable of replicating itself, and sometimes both are not.

A "totipotent stem cell" has the ability to give rise to all types of cells, including the three germ layers (mesoderm, endoderm, and ectoderm) from which all cells of the body arise.

A "pluripotent stem cell" has the ability to give rise to types of cells that develop from said three germ layers. An "embryonic stem cell" is derived from a group of cells called the inner cell mass, which is part of the early (4-5 day) embryo called the blastocyst. Embryonic stem cells are pluripotent.

An "embryonic germ cell" is derived from fetal tissue. Specifically, they are isolated from the primordial germ cells of the gonadal ridge of the 5-10-week fetus. Embryonic germ cells are pluripotent.

An "adult stem cell" is an undifferentiated (unspecialized) cell that occurs in a differentiated (specialized) tissue, renews itself, and becomes specialized to yield all of the specialized cell types of the tissue from which it originated. However, under certain conditions some adult stem cells are also capable to specialize into cells of a type of tissue they do not originate from. Adult stem cells are capable of making identical copies of of themselves for the lifetime of the organism. Adult stem cells usually divide to generate progenitor or precursor cells, which then differentiate or develop into "mature" cell types that have characteristic shapes and specialized functions. They do not replicate indefinitely in culture.

A "progenitor or precursor cell" occurs in fetal or adult tissues and is partially specialized; it divides and gives rise to differentiated cells. When a progenitor or precursor cell divides, it can form more progenitor or precursor cells or it can form two specialized cells. The difference between progenitor cells and stem cells is gradual. Therefore it is difficult to provide a precise definition. However, as a rule a stem cell is more "potent" and less specialized than a progenitor.

"Phenotype", in this context, is understood as incorporating the observable characteristics of an organism or cell. This includes characteristics that are not visible to the eye but can be observed by employing biochemical assays, such as protein expression. In contrast to the phenotype the genotype is an organisms' genetic composition. The phenotype results from the specific interaction of an organism defined by its genotype with the environment. In this context the epigenetic composition of the genome, i.e. its methylation status, is not understood as part of the phenotype.

The term "microarray" refers broadly to both 'DNA microarrays' and 'DNA chip(s)' as recognized in the art, encompasses all art-recognized solid supports, and encompasses all methods for affixing nucleic acid molecules thereto or synthesis of nucleic acids thereon.

All references cited herein are incorporated by reference in their entirety.

The subject of this invention is a method to monitor the differentiation of at least one cell from a state 1 into a state 2, characterized in that the following steps are carried out 1) the cytosine methylation pattern of a DNA sample taken from at least one prototype cell at said state 1 is determined or provided, 2) the cytosine methylation pattern of a DNA sample taken from at least one prototype cell at said state 2 is determined or provided, 3) at least one cell at said state 1 is exposed to conditions, which are expected to convert a cell at said state 1 into a cell at said state 2, 4) measuring the cytosine methylation pattern in a DNA sample taken from said cell or cells that were exposed to conditions, which are expected to convert a cell at said state 1 into a cell at said state 2, 5) comparing the cytosine methylation pattern measured in step 4) with the cytosine methylation patterns determined or provided in steps 1) and 2), and 6) concluding whether the conversion of said cell or cells that were exposed to conditions, which are expected to convert a cell at said state 1 into a cell at said state 2, took place, or whether it was complete and/or effective.

### More detailed description of the invention:

The method which is subject of this invention consists of several steps.

The first step of the method is to determine or provide a cytosine methylation pattern of a DNA sample taken from at least one prototype cell at a state 1 and the second step is to determine or provide a cytosine methylation pattern of a DNA sample taken from at least one prototype cell at a state 2. "To determine" in this context is meant to comprise the identification and/or detection of a pattern that can be correlated with said state of a cell. This is independent from the question whether said pattern has been known previously or not. The term "provided" is meant to also explicitly include the use of patterns already well known to the public and documented.

For example, if the methylation pattern of cytosine residues of a sufficient number of genes in haematopoietic stem cells is known to unambiguously identify their state of differentiation this information will be used in step 1 and/or step 2 if said prototype cell of interest is the haematopoietic stem cell.

If the cytosine methylation pattern of a specific prototype of cell and state of interest is not sufficiently well characterized it has to be determined.

Methods on how to determine such a methylation pattern are described in the literature (for example: Adorjan et al. (2002) Tumor class prediction and discovery by microarray-based DNA methylation analysis. Nucleic Acids Res. 30 (5), e21) and in for example patents WO 99/28498 (Olek et al.), WO 00/70090 (Laird et al.), U.S. Patent 6,265,171 (Herman and Baylin), U.S. Patent 6,251,594 (Gonzalgo et al.).

Generally, two different scenarios are possible, either the relevant marker genes are already known or they are unknown. To determine a specific methylation pattern of a DNA sample taken from at prototype cell at a state 1, for which no marker genes are known, those have to be discovered first. This step of identifying the markers is understood to be included in the term "determination" of the steps 1 and/or 2 whenever this proves to be necessary. Particularly where sequence data is unavailable the marker discovery is preferably done by applying one or several of the following techniques:
Differential methylation hybridization (DMH)
   (Huang et al. (1999) Methylation profiling of CpG islands in human breast cancer cells. Hum Mol Genet. 8, 459-70)
Restriction landmark genomic scanning (RLGS)
   (Hatada et al. (1991) A Genomic Scanning Method for Higher Organisms Using Restriction Sites as Landmarks. PNAS 88, 9523-9527; Hatada et al. (1993) A new imprinted gene cloned by a methylation-sensitive genome scanning method. Nucleic Acids Res. 21, 5577-5582)
Methylation sensitive arbitrarily primed PCR (AP-PCR)
   (Gonzalgo et al. (1997) Identification and characterization of differentially methylated regions of genomic DNA by methylation-sensitive arbitrarily primed PCR. Cancer Res. 57, 594-599)
Methylated CpG island amplification (MCA)
   (Toyota et. al. (1999) Identification of differentially methylated sequences in colorectal cancer by methylated CpG island amplification. Cancer Res. 59, 2307-2312; Toyota, M and Issa, D (2002) Methylated CpG island amplification for methylation analysis and cloning differentially methylated sequences. Methods Mol Biol 200, 101-110)

All of the methods mentioned above may be used to identify and validate suitable candidate CpG positions for use as markers.

Knowing the marker genes specific for the state of interest, the methylation pattern of said DNA sample can be determined for example by the use of methylation specific restriction endonucleases, which are capable of catalytically hydrolyzing (digesting) DNA at and/or upon recognition of specific sequences, usually between 4 to 8 bases in length. Methylation specific restriction endonucleases are characterized in only digesting the DNA when a specific methylation state is present in the recognition sequence. The digested DNA fragments are preferably separated on the basis of size (e.g. by gel electrophoresis). The methylation status of the sequence is thereby deduced. Preferably a post-digest PCR amplification step is added wherein two primers on either side of the restriction site are used to amplify the digested DNA. PCR products are detectable only in the case of digestion not occurring.

Another preferred method of methylation analysis of markers is the modification of CpGs followed by sequence analysis. A preferred chemical reagent that can be used to distinguish between methylated and non methylated CpG positions is hydrazine, which cleaves the nucleic acid.

However, a more preferred method to distinguish the methylated and unmethylated cytosines is the bisulfite treatment, preferably followed by alkaline hydrolysis (Olek et al. (1996) A modified and improved method for bisulfite based cytosine methylation analysis. Nucleic Acids Res. 24, 5064-5066). The treated DNA can be analyzed by conventional molecular biology techniques, for example, PCR amplification, sequencing and/or detection oligonucleotide hybridization.

MSP (Methylation Specific PCR) is a preferred method to determine whether a CpG site is methylated or unmethylated, which uses methylation sensitive primers. The DNA of interest is treated such that methylated and non methylated cytosines are differentially modified in a manner discernable by their hybridization behavior. This can preferably be done with bisulfite treatment (see above). PCR primers specific to either the previously methylated CpG or the previously unmethylated CpG are used in a PCR amplification step. Products of the amplification reaction are then detected, allowing for the deduction of the methylation status of the CpG position within the genomic DNA (U.S. Patent 6,265,171 to Herman and Baylin).

More preferred methods for the analysis of bisulfite treated DNA include the use of primer extension as described by Gonzalgo et. al. (U.S. Patent 6,251,594) and the use of real time PCR based methods.

All of the methods mentioned above may be used to determine the methylation patterns of DNA samples which are specific for DNA from a cell at said stage 1 or said stage 2.

The third step of the method is to expose at least one cell at said state 1 to conditions, which are expected to convert a cell at said state 1 into a cell at said state 2.

This can be done in plenty of ways. Any treatment used in cell culture with the aim to induce cell proliferation, cell differentiation, cell dedifferentiation or even a-poptosis is in this context understood as expected to convert a cell at one state into a cell at another state. For example, growing cells in a medium that contains a growth hormone is herein understood as exposure of the cells to conditions that are expected to convert a cell at state 1 into a cell at state 2. For example, hES (human embryonic stem) cells can be derived and maintained in an undifferentiated, pluripotent state in cultures. Without a layer of feeder cells, cultured hES cells maintain their pluripotency for brief periods only. In this context a cell at state 1 would be a freshly isolated, pluripotent hES cell and a cell at state 2 would be a hES cell that has lost its pluripotency due to an extended culturing period without adding a layer of feeder cells. In this case the culturing itself is the condition exposed on the cells at state 1 to convert them in a state 2.
In another example, if said cell at state 1 is the freshly cultured, pluripotent hES and the conditions said cell is exposed to, which are expected to convert said cell, are the culturing and addition of said human fetal fibroblasts as feeders, thereby retaining the cell's undifferentiated state and pluripotency, the cell at state 2 is a hES cell cultured with the feeding layer, which still is pluripotent.

For both cases it would be required to find CpG positions that are specifically methylated or specifically unmethylated depending on whether the cell was freshly cultured or not and to find CpG positions that are specifically methylated or specifically unmethylated depending on whether the cell is pluripotent or not. In the second example additional markers would be required that are specifically methylated depending upon whether the cell has been cultured with a feeding layer or without.

The fourth step of the method consists of measuring the cytosine methylation pattern in a DNA sample taken from said cell or cells that were exposed to conditions, which are expected to convert a cell at said state 1 into a cell at said state 2. For example, in the first experiment (example above) one would measure the cytosine methylation pattern of hES cells that have been exposed to culturing medium for a couple of days. In the second experiment one would measure the cytosine methylation pattern of hES cells that have been exposed to the culturing conditions containing human fetal fibroblasts as feeders.

A number of methods for measuring the cytosine methylation pattern in a DNA sample exist and they are well documented. Any method, e.g., the sequencing technique, MSP-PCR technique, southern blotting, or oligo microarray hybridization technique, may be used to identify methylation patterns. It should be noted that techniques useful in the present invention for obtaining information on methylation are not limited to the above-mentioned techniques. Any technique may be used as long as information on methylation can be obtained with it. Some of the preferred methods are described here:

Prior to every methylation detection assay DNA of the sample to be analyzed has to be isolated. DNA extraction may be by means that are standard to one skilled in the art, these include steps such as the use of detergent lysates, sonification and vortexing with glass beads, followed by ethanol precipitation. Once the nucleic acids have been extracted the genomic double stranded DNA is used in the analysis.

As in this step of the method the relevant marker genes, specific for the state of interest, are already known, the methylation pattern of said DNA sample can be determined, for example, by the use of methylation specific restriction endonucleases. They are characterized by only digesting the DNA when a specific methylation state is present in the recognition sequence. The digested DNA fragments are preferably separated on the basis of size (e.g. by gel electrophoresis) and can be detected more specifically with the southern blot technique. The methylation status of the sequence is thereby deduced.

Preferably a post digest PCR amplification step is added prior to the separation step wherein two primers on either side of the digest site are used to amplify the digested DNA. PCR products are detectable only in the case of digestion not occurring.

A number of preferred methods of methylation analysis are based on the modification step that is performed prior to detection of the specific site. This step consists of the modification of CpGs in a way that allows to differentiate between previously methylated and non-methylated cytosines.

The genomic DNA sample of interest is treated in such a manner that cytosine bases which are unmethylated at the C5-position are converted to uracil, thymine, or another base which is dissimilar to cytosine in terms of hybridization behavior, whereas 5-methylcytosine is not. This will be understood as 'pretreatment' hereinafter. For the purpose of this invention "genomic DNA" is understood as the untreated DNA which is extracted from the sample of interest.

The above described treatment of genomic DNA is preferably carried out with bisulfite (hydrogen sulfite, disulfite) and subsequent alkaline hydrolysis (Olek, A et al. (1996) A modified and improved method for bisulfite based cytosine methylation analysis. Nucleic Acids Res. 24, 5064-5066) which results in a conversion of non-methylated cytosine nucleobases to uracil which is dissimilar to cytosine in terms of base pairing behavior.

The treated DNA can be analyzed by conventional molecular biology techniques. Fragments of the pretreated DNA are amplified, using sets of primer oligonucleotides and a, preferably heat-stable polymerase. Because of statistical and practical considerations, preferably more than one different fragment having a length of 100 - 2000 base pairs are amplified. The amplification of several DNA segments can be carried out simultaneously in one and the same reaction vessel. Usually, the amplification is carried out by means of a polymerase chain reaction (PCR). The amplificate DNA is then analyzed using conventional techniques:

As this sequence conversion can lead to the methylation status specific creation of new restriction sites and also to the methylation status specific retention of preexisting sites, the methylation status can be detected using enzymatic digestion (Xiong, Z and Laird, PW (1997) COBRA: a sensitive and quantitative methylation assay. Nucleic Acids Res. 25, 2535-2534).

Another preferred method to detect the CpG positions that were methylated or unmethylated prior to the treatment with bisulfite and to determine the methylation pattern is to sequence the converted DNA and compare it to the sequence of the unconverted DNA (Grunau C, Clark SJ, Rosenthal A (2001) Bisulfite genomic sequencing: systematic investigation of critical experimental parameters. Nucleic Acids Res. 29, E65).

Another preferred method to detect the methylation status of specific CpG positions within the bisulfite treated nucleic acid marker uses methylation specific primer oligonucleotides (MSP). This technique has been described in U.S. Patent 6,265,171 to Herman and Baylin. The use of methylation status specific primers for the amplification of bisulfite treated DNA allows the differentiation between methylated and unmethylated nucleic acids. MSP primer pairs contain at least one primer which hybridizes to a bisulfite treated CpG dinucleotide.

PCR primers specific to either the prior to treatment methylated or prior to treatment unmethylated CpG sites are used in a PCR amplification step. Products of the amplification reaction are then detected, allowing for the deduction of the methylation status of the CpG position within the genomic DNA. (U.S. Patent 6,265,171 (Herman & Baylin))

More preferred methods for the analysis of bisulfite treated DNA include the use of primer extension as described by Gonzalgo et. al. (U.S. Patent 6,251,594) and the use of real time PCR based methods.

The fragments obtained by means of the amplification can carry a directly or indirectly detectable label. Preferred are labels in the form of fluorescence labels, radionuclides, or detachable molecule fragments having a typical mass which can be detected in a mass spectrometer. Wherein said labels are mass labels it is preferred that the labeled amplificates have a single positive or negative net charge for better detectability in the mass spectrometer. The detection may be carried out and visualized by means of matrix assisted laser desorption/ionization mass spectrometry (MALDI) or using electron spray mass spectrometry (ESI).

The amplificates obtained are analyzed in order to ascertain the methylation status of the CpG dinucleotides prior to the treatment.

Wherein the amplificates were obtained by means of MSP amplification the presence or absence of an amplificate is in itself indicative of the methylation state of the CpG positions covered by the primer, according to the base sequences of said primer.

Amplificates obtained by means of both standard and methylation specific PCR may be further analyzed by means of hybridization based methods such as, but not limited to, array technology and probe based technologies as well as by means of techniques such as sequencing and template directed extension.

Preferably, the synthesized amplificates are subsequently hybridized to an array or a set of oligonucleotides and/or PNA probes. In this context, the hybridization takes place in the manner described in the following. The set of probes used during the hybridization is preferably composed of at least 2 oligonucleotides or PNA-oligomers. In the process, the amplificates serve as probes which hybridize to oligonucleotides immobilized to a solid phase. The non-hybridized fragments are subsequently removed. Said oligonucleotides contain at least one base sequence having a length of at least 9 nucleotides which is reverse complementary or identical to a segment of the base sequences, which contains the known marker CpGs. In a preferred embodiment said dinucleotide is present in the central third of the oligomer. For example, wherein the oligomer comprises one CG dinucleotide, said dinucleotide is preferably the 5th to 9th nucleotide from the 5'-end of a 13-mer.

Finally, the hybridized amplificates are detected. In this context, it is preferred that labels attached to the amplificates are identifiable at each position of the solid phase at which an oligonucleotide sequence is located.

In a further embodiment of the method, the methylation status of the CpG positions (prior to treatment) may be ascertained by means of oligonucleotide probes that are hybridized to the bisulfite treated DNA concurrently with the PCR amplification primers (wherein said primers may either be methylation specific or standard).

A particularly preferred method is the use of fluorescence-based Real Time Quantitative PCR (Heid CA et al. (1996) Real Time quantitative PCR. Genome Res. 6: 986-994) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which is designed to hybridize to a CpG rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment it is required that the probe be methylation specific, as described in U.S. 6,331,393 to Laird (hereby incorporated by reference) also known as the Methyl Light assay. Variations on the TaqMan™ detection methodology that are also suitable for use with the described invention include the use of dual probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids is the use of blocker oligonucleotides. The use of such oligonucleotides has been described (Yu D, Mukai M, Liu Q, Steinman C (1997) Specific inhibition of PCR by non-extendable oligonucleotides using a 5' to 3' exonuclease-deficient DNA polymerase. BioTechniques 23: 714-720.) Blocking probe oligonucleotides are hybridized to the bisulfite treated nucleic acid concurrently with the PCR primers. PCR amplification of the nucleic acid is terminated at the 5' position of the blocking probe, thereby amplification of a nucleic acid is suppressed wherein the complementary sequence to the blocking probe is present. The probes may be designed to hybridize to the bisulfite treated nucleic acid in a methylation status specific manner. For example, for detection of methylated nucleic acids within a population of unmethylated nucleic acids suppression of the amplification of nucleic acids which are unmethylated at the position in question would be carried out by the use of blocking probes comprising a 'CA' at the position in question, as opposed to a 'CG'.

In a further preferred embodiment of the method the methylation analysis is carried out by the use of template directed oligonucleotide extension, such as MS-SNuPE (Gonzalgo ML and Jones PA (1997) Rapid quantitation of methylation differences at specific sites using methylation-sensitive single nucleotide primer extension (Ms-SNuPE). Nucleic Acids Res. 25, 2529-2531).

The fifth step of the method consists of comparing the cytosine methylation pattern measured in step 4 with the cytosine methylation patterns determined or provided in steps 1 and 2. For this the methods described above are conducted and the results are compared. The results can be compared in any usual manner, as will be obvious to a person skilled in the art. For example the results can be analyzed and compared numerically, with or without the use of statistical methods. Results can also be compared by a simplified match or mismatch of pattern decision. Every single CpG site's methylation state can be compared with its one or several counterparts or a complete pattern can be compared with the complete pattern of its one or several counterparts.

The sixth step of the method consists of concluding whether the conversion of said cell or cells that were exposed to said conditions, which are expected to convert a cell of said state 1 into a cell of said state 2, took place or whether it was complete and/or effective. In this step the results of the previous steps are interpreted and by this transformed into a useful bit of information regarding, for example, the efficiency of said conditions.

For example, if the experiment would address the transfer of chondrocytes from a state 1, defined as having been isolated and growing in culture for less than a day, into a state 2, defined as a dedifferentiated chondrocyte cell, and given the respective specific methylation patterns were known, step 3 would consist of culturing the chondrocytes with the purpose of dedifferentiating them (for specific conditions see example 1), step four would consist of measuring the methylation pattern of the DNA of at least one cell of the culture expected to be dedifferentiated and step five would consist of the comparison of said measured pattern with the pattern that was already known before. In step six it is concluded whether the expected conversion did take place. It can also be concluded from comparing the results of a number of state specific CpG positions if the cell of interest might have reached a state somewhere in between state 1 and state 2 and hence whether or not the transition was complete or not.

If a quantitative assessment had been performed on a cell culture, as in a number of cells, it can be concluded whether the conversion of a cell culture at state 1 into a cell culture at state 2 has been complete or not, that is whether all cells in the culture have been converted or only some.

Furthermore the present invention concerns said method described above wherein one of said cell states is characterized as being more specialized and/or further differentiated than the other.

It is also preferred that one of said cell states is characterized as a cell fully differentiated and biologically functioning. In this context a cell that is biologically functioning is understood as a cell performing the exact metabolic mechanisms in the manner required for the specific cell type in question, without displaying any characteristics non-typical for the specific cell type at this particular differentiation state.

In a further aspect, the present invention provides a method to monitor the differentiation of at least one cell from a state 1 into a state 2, characterized in that one of said cell states is characterized as being a cell of the smooth muscle, striated muscle, skeletal muscle, cardiac muscle, connective tissue, bone, cartilage, kidney, urogenital system, adrenal cortex, heart, blood vessels, bone marrow, thymus, thyroid, parathyroid glands, larynx, trachea, lung, lining of the respiratory tract, urinary bladder, vagina, urethra, gastrointestinal organs, liver, pancreas, gut epithelium, the lining of the gastrointestinal tract, brain, skin, eye, ear, connective tissue of the head and face, neural epithelium, pituitary gland, embryonic ganglia, stratified squamous epithelium, adrenal medulla or lymphatic tissue or a haematopoietic cell, astrocyte, oligodendrocyte, myocyte, adipocyte, chondrocyte, osteocyte, cardiomyocyte, neuron, keratinocyte, bone marrow stromal cell, thymic stromal cell, hepatocyte, haematopoietic cell, cholangiocyte, red blood cell or white blood cell.

A further embodiment of the invention is said method as described before wherein a cell at said state 1 is a stem cell and/or progenitor cell.

A further embodiment of the invention is said method as described before wherein a cell at said state 1 is a fetal tissue germ cell, a primordial germ cell, an embryonic stem cell, a cell of the embryoid body, a cell from the blastocyst inner cell mass (ICM), or an adult stem cell.

A further embodiment of the invention is said method as described before wherein a cell at state 1 is a haematopoietic stem cell (HSC), mesenchymal stem cell (MSC), neural stem cell (NSC), human central nervous system stem cell (hCNS-SC) or a stem cell isolated from a stromal vascular cell fraction of processed lipoaspirate.

A further embodiment of the invention is said method as described before wherein a cell at state 1 or state 2 is a haematopoietic progenitor cell, myeloid progenitor cell, lymphoid progenitor cell, mesenchymal progenitor cell, a nestin-positive islet-derived progenitor cell or neural progenitor cell.

A further embodiment of the invention is said method as described before wherein a cell at state 1 is a cell of the endoderm, mesoderm or ectoderm.

A further embodiment of the invention is said method as described before wherein a cell at state 1 is an ectoderm derived cell and a cell at state 2 is a cell of the brain, skin, eye, ear, connective tissue of the head and face, neural epithelium, pituitary gland, embryonic ganglia, stratified squamous epithelium or adrenal medulla.

A further embodiment of the invention is said method wherein a cell at said state 1 is an endoderm derived cell and a cell at said state 2 is a cell of the thymus, thyroid, parathyroid glands, larynx, trachea, lung, lining of the respiratory tract, urinary bladder, vagina, urethra, gastrointestinal organs, liver, pancreas, gut epithelium or the lining of the gastrointestinal tract.

A further embodiment of the invention is said method wherein a cell at said state 1 is a mesoderm derived cell and a cell at said state 2 is a cell of the smooth muscle, striated muscle, skeletal muscle, cardiac muscle, connective tissue, bone, cartilage, kidney, urogenital system, adrenal cortex, heart, blood vessels, bone marrow or lymphatic tissue or a haematopoietic cell.

A further embodiment of the invention is said method wherein a cell at said state 1 is a haematopoetic stem cell and a cell at said state 2 is a haematopoietic progenitor cell, hepatocyte, cholangiocyte, red blood cell or white blood cell.

A further embodiment of the invention is said method wherein a cell at said state 1 is a mesenchymal stem cell and a cell at said state 2 is a myocyte, adipocyte, chondrocyte, osteocyte, cardiomyocyte, neuron, bone marrow stromal cell or thymic stromal cell.

A further embodiment of the invention is said method wherein a cell at said state 1 is a neural stem cell or a human central nervous system stem cell and a cell at said state 2 is a muscle cell, neuron cell, astrocyte or oligodendrocyte.

A further embodiment of the invention is said method wherein a cell at said state 1 is isolated from a stromal vascular cell fraction of processed lipoaspirate and a cell at said state 2 is an adipocyte precursor, osteocyte precursor, chondrocyte precursor or myocyte precursor cell.

A further embodiment of the invention is said method wherein a cell at said state 2 is an endocrine pancreatic cell. It is especially preferred that said pancreatic cell produces insulin. Furthermore it is especially preferred that said pancreatic cell produces insulin in a glucose responsive manner.

A further embodiment of the invention is said method wherein a cell at said state 1 is a cell from the blastocyst inner cell mass and a cell at said state 2 is an endocrine pancreatic cell. It is especially preferred that said pancreatic cell produces insulin. Furthermore it is especially preferred that said pancreatic cell produces insulin in a glucose responsive manner.

A further embodiment of the invention is said method wherein a cell at said state 1 is a nestin-positive islet-derived progenitor cell and a cell at said state 2 is an endocrine pancreatic cell or a hepatic cell. It is especially preferred that said pancreatic cell produces insulin. Furthermore it is especially preferred that said pancreatic cell produces insulin in a glucose responsive manner.

It is preferred that a cell at one of said states is a chondrocyte. It is especially preferred that said chondrocytes are isolated from a human cartilage sample.

It is also preferred that cells at said state 1 are fully differentiated chondrocytes and cells at state 2 are dedifferentiated and/or expanded. It is especially preferred that said chondrocytes are isolated from a human cartilage sample.

A further embodiment of the invention is said method wherein a cell at one of said states is a circulatory skeletal blood cell and said cell at the other state is an adipocyte or an osteocyte.

A further embodiment of the invention is said method wherein a cell at one of said states is an angioblast cell from the bone marrow and said cell at the other state is a cell of a newly formed blood vessel or a mature endothelial cell.

Said method is a preferred embodiment of the invention when the DNA sample is taken from a source such as a cell culture or a tissue culture.

Said method wherein said conditions are characterized as allowing the cells and/or cell cultures to grow on scaffolds or otherwise in 3 dimensional conditions is another embodiment of the invention.

In a further aspect, the present invention is characterized as a method wherein said conditions include the feeding of said cells on one or several reduced media or supplemented media.

It is a preferred embodiment of the present invention, that said supplemented media contain growth or differentiation inducing factors, natural serums, natural extracts, synthetic supplements, recombinant growth factors or chemicals, which induce growth or differentiation, or a mixture of any of those.

It is a preferred embodiment of the present invention, that said conditions include the feeding of cells on a feeder cell layer.

Preferably said method's conditions are characterized by a specified temperature, humidity, light, electrical field, magnetic field, O2, N2 and/or CO2 concentration.

In another embodiment of said invention said conditions, which are expected to convert a cell from a state 1 into a state 2 , include the treatment of said cells at state 1 with an effective amount of a agent, which is able to modify said cell's DNA methylation status.

It is preferred that said agent belongs to the group of 5-aza-2'-deoxycytidine, Trichostatin A, lankacidin, benzenamine, cyclohexane acetic acid, blue platinum uracil, methyl 13-hydroxy-15-oxo-kaurenoate, sulfonium, euphornin D, octadecylphosphoryl choline, gnidimarcin, and aspiculamycin HCL.

In another embodiment it is especially preferred that said agent belongs to the group consisting of inhibitors of DNA methylation and histone deacetylation, topoisomerase II, and DNA synthesis.

It is another embodiment of this invention to use said method as described herein on specific purposes. Some of those will be explicitly mentioned, but the list of potential uses is not meant to be limiting. It is the main purpose of this invention to provide a quality control tool to assess the quality of the tissue engineering process. It is understood that the whole process - from the first steps (of first characterizing and isolating cells of a quality high enough for the differentiation and engineering process, such as adult stem cells, for example) to the final step (of transplanting tissue engineered material into a patient, for example) - will benefit from a tool that allows to monitor the process by assessing the differentiation state of a cell by a fast and simple test on a limited amount of available DNA.

Therefore it is a preferred embodiment of the invention to use said method for improving the tissue engineering process.

It is also a preferred embodiment of the invention to use said method for monitoring a cell differentiation process.

It is especially preferred to use said method for monitoring the differentiation of cell lines derived from in vitro sources and cell lines derived from in vivo and/or autopsy sources.

It is also especially preferred to use said method for monitoring a process comprising several steps of transition of a cell from one state into another.

Furthermore it is preferred to use said method for validation of engineered tissue cells.

In a further aspect, the present invention provides a method that is characterized in its possible use to quality assess the final tissue engineered product. It is of crucial importance to the patient that the engineered tissue is homogenous and does not contain any undifferentiated cells.

It is therefore a preferred embodiment of the invention to use said method to distinguish between omnipotent cells and more differentiated cells.

It is also a preferred embodiment of the invention to use said method for ensuring homogeneity of the cultured cells.

It is especially preferred to use said method for detecting contamination of differentiated cells or of engineered tissue with uncontrolled proliferating cells, such as progenitor or stem cells.

In a further aspect, the present invention provides a method that is characterized in its potential to identify the individual source an engineered tissue is derived from. When offering individualized products, that are developed from autologous sources, before feeding back a tissue engineered product into a patient it needs to be ensured that the product was indeed developed from his own cells.

It is therefore a preferred embodiment of the invention to use said method for identification of a tissue's cell of origin.

Furthermore it is especially preferred to use said method for ensuring that the engineered tissue is derived from a specifically defined cell source.

Finally it is a preferred embodiment of the invention to use said method for post surgery evaluation of the development of tissue transplanted into a patient.

The example described below is meant to explain and enable the invention.

### Example:

### Methylation analysis to improve differentiation conditions of chondrocytes in culture and to improve the chondrocytes growth.

For comparative analysis chondrocyte samples of at least three individuals without known history of joint problems are taken from a tissue library. These are compared with cartilage tissue samples taken from patients that had joint replacement surgery with different disease indications.

### Isolation

Chondrocyte cells are isolated by incubating the cartilage tissue sample for a period of 22 hours at 37°C in 0.15% type II collagenase, resuspending it in Dulbeccos modified Eagle medium (DMEM: detailed information about it can be found for example at :
http://methdb.igh.cnrs.fr/cgrunau/cell_lines/DMEM.pdf). Said medium ideally contains recombinant or synthetic growth factors or growth factors isolated from serum taken from autologous sources. Alternatively the medium may contain FBS (fetal bovine serum).

After isolation and purification of the chondrocytes each sample is divided into at least four aliquots. One of these aliquots is frozen directly after purification or, alternatively, instantly prepared for methylation analysis.

The remaining samples are cultured according to the protocols described herein, similar ones or variations thereof with the aim of proliferation and re-differentiation of these chondrocytes.

### De-differentiation and growth

Chondrocytes of the remaining samples are plated in tissue culture flasks at a density of 104 cells/cm2 and cultured at 37°C/5% CO2. After 10 days, the cells are subconfluent and are dissolved from the bottom of the flask with 0.25% trypsin in order to plate a second time at a density of 5 x 103 cells/cm2. After ca. another week the confluent cells are treated with trypsin again and pelleted. Those cultures, also called P2-cultures are resuspended and re-differentiated in SFM or SSM medium.

### Re-differentiation

For re-differentiation, chondrocytes that are growing at a cell density of 5 x 105 cells per ml are centrifuged for 15 sec at 7500 rpm in 0.5 ml medium. Those pelleted cultures are placed in a 3D orbital shaker and grown at 30 rpm at 37°C/5% CO2 for about 2 weeks (Jakob et al. (2001) Specific growth factors during the expansion and redifferentiation of adult human articular chondrocytes enhance chondrogenesis and cartilaginous tissue formation in vitro. J Cell Biochem 81, 368-77).

At several specific time points during growth and differentiation cell samples are prepared for methylation analysis.

### DNA purification

For this purpose genomic DNA is isolated and purified from said cell samples according to the manufacturers guidelines given in the QIAamp DNA minikit.

### Bisulfite treatment

The isolated and purified DNA is digested with MssI and treated with bisulfite as described (Olek A, Oswald J and Walter J (1996) A modified and improved method for bisulfite based cytosine methylation analysis. Nucleic Acids Res. 24, 5064-66).

### Amplification for the microarray (chip) based analysis

The bisulfite treated and successfully converted DNA is amplified via PCR and with the use of a specifically improved oligonucleotide-design method (Clark und Frommer (1997) Bisulfite genomic sequencing of methylated cytosines. In Taylor, G.R. (ed.) Laboratory Methods for the detection of Mutations and Polymorphisms in DNA. CRC Press, Boca Raton, FL, pp 151-61).

### Microarray procedure

Oligonucleotides with a C6-amino modification at the 5'-end are spotted with 4-fold redundancy on activated glass slides (Golub et al. (1999) Molecular classification of cancer: class discovery and class prediction by gene expression monitoring. Science 286, 531-557). For each analysed CpG position two oligonucleotides, one containing a CG, the other one containing a TG, reflecting the methylated and non-methylated status of the CpG dinucleotides, are spotted and immobilized on the glass array. Oligonucleotides are designed such that they match only the bisulfite-modified DNA fragments; this is important to exclude signals arising from incomplete bisulfite conversion. The oligonucleotide microarrays representing up to 235 CpG sites are hybridized with a combination of up to 56 Cy5-labelled PCR fragments as described earlier (Chen D, Yan Z, Cole DL and Srivatsa GS (1999) Analysis of (n-1)mer deletion sequences in synthetic oligodesoxyribonucleotides by hybridization to an immobilized probe array. Nucleic Acids Res. 27: 389-395). Subsequently, the fluorescent images of the hybridized slides are obtained using a GenePix 4000 microarray scanner (Axon Instruments). Hybridization experiments are repeated at least three times for each sample.

### Classification of differentially developed chondrocytes:

The CpG sites analyzed with the purpose of classifying the differentiation state of chondrocytes are located in the regulatory parts of one or several genes of the group comprising : Interleukin-1b, BMP-2/9, TGF-beta, FGF-2, Indian Hedgehog, Syndecan-3, PNCA, CollagenI/CollagenII, Aggrecan/CDRAP and Versican, Collagen XI, Collagen X, A-11, Viglin, COMB, TRAX/ Translin, Matrilin-I, Fibromodulin, Epiphycan, Decorin, Biglycan, Sox-5, Sox-6, Sox-9, PTHrP, Chondroadherin, Annexin VI, Alkaline Phosphatase, GDF5, Noggin, Caspase3, Erk1/2. MEK/Erk, pMAPK38, Tyrosine Kinase, Vinculin, ID1, Cyclin D1, C-jun, JunD, NFkB.

### Statistical methods

For class prediction (in order to differentiate between tissue development stages) a support vector machine (SVM) is used on a set of selected CpG sites. First the CpG sites for a given separation task are ranked by the significance of the difference between the two class means. The significance of each CpG is estimated by a two sample t-test. Then a SVM is trained on the most significant CpG positions, where the optimal number of CpG sites depends on the complexity of the separation task. The implementation of the SVM used the Sequential Minimal Optimization algorithm to find the 1-norm soft margin separating hyperplane (Christianini N and Shawe-Taylor J (2000) An Introduction to Support Vector Machines and Other Kernel-Based Learning Methods. Cambridge University Press, Cambridge, UK, pp 137-144).

To apply an additional independent data validation method direct bisulfite sequencing reactions and/or Real Time PCR are performed for those CpGs that seem to be significant based on the interpretation of chip based and statistical validation data.

The most significant CpGs found allow an unambiguous discrimination of at least 4 different differentiation stages of chondrocytes, being:
- Sickness-related, activated chondrocytes, as clear indication of artheosclerotic illness.
- healthy, biopsy-taken, completely differentiated and growth inhibited chondrocytes.
- dedifferentiated, proliferating chondrocyte precursors
- correct as well as incorrect re-differentiated, in vitro grown, growth inhibited chondrocytes.

## Claims

1. A method to monitor the differentiation of at least one cell from a state 1 into a state 2, **characterized in that** the following steps are carried out
a) the cytosine methylation pattern of a DNA sample taken from at least one prototype cell at the state 1 is determined or provided,
b) the cytosine methylation pattern of a DNA sample taken from at least one prototype cell at the state 2 is determined or provided,
c) at least one cell at the state 1 is exposed to conditions, which are expected to convert a cell at said state 1 into a cell at said state 2,
d) determining the cytosine methylation pattern in a DNA sample taken from said cell or cells that were exposed to conditions, which are expected to convert a cell at said state 1 into a cell at said state 2,
e) comparing the cytosine methylation pattern measured in step d) with the cytosine methylation patterns determined or provided in step a) and b) and
f) concluding whether the conversion of said cell or cells that were exposed to conditions, which are expected to convert a cell at said state 1 into a cell at said state 2 took place, was complete and/or effective.

2. A method according to claim 1, wherein one of said cell states is characterized as being more specialized and/or further differentiated than the other.

3. A method according to claim 1, wherein one of said cell states is characterized as a cell fully differentiated and biologically functioning.

4. A method according to claims 1 and 2, wherein one of said cell states is characterized as being a cell of the smooth muscle, striated muscle, skeletal muscle, cardiac muscle, connective tissue, bone, cartilage, kidney, urogenital system, adrenal cortex, heart, blood vessels, bone marrow, thymus, thyroid, parathyroid glands, larynx, trachea, lung, lining of the respiratory tract, urinary bladder, vagina, urethra, gastrointestinal organs, liver, pancreas, gut epithelium, the lining of the gastrointestinal tract, brain, skin, eye, ear, connective tissue of the head and face, neural epithelium, pituitary gland, embryonic ganglia, stratified squamous epithelium, adrenal medulla or lymphatic tissue or a haematopoietic cell, astrocyte, oligodendrocyte, myocyte, adipocyte, chondrocyte, osteocyte, cardiomyocyte, neuron, keratinocyte, bone marrow stromal cell, thymic stromal cell, hepatocyte, haematopoietic cell, cholangiocyte, red blood cell or white blood cell.

5. A method according to claim 1, wherein a cell at said state 1 is a stem cell and/or progenitor cell.

6. A method according to claim 1, wherein a cell at said state 1 is a fetal tissue germ cell, a primordial germ cell, an embryonic stem cell, a cell of the embryoid body, a cell from the blastocyst inner cell mass (ICM), or an adult stem cell.

7. A method according to claim 1, wherein a cell at state 1 is a haematopoietic stem cell (HSC), mesenchymal stem cell (MSC), neural stem cell (NSC), human central nervous system stem cell (hCNS-SC) or a stem cell isolated from a stromal vascular cell fraction of processed lipoaspirate.

8. A method according to claim 1, wherein a cell at state 1 or state 2 is a haematopoietic progenitor cell, myeloid progenitor cell, lymphoid progenitor cell, mesenchymal progenitor cell, a nestin-positive islet-derived progenitor cell or neural progenitor cell.

9. A method according to claim1, wherein a cell at state 1 is a cell of the endoderm, mesoderm or ectoderm.

10. A method according to claim 1, wherein a cell at state 1 is an ectoderm derived cell and a cell at state 2 is a cell of the brain, skin, eye, ear, connective tissue of the head and face, neural epithelium, pituitary gland, embryonic ganglia, stratified squamous epithelium or adrenal medulla.

11. A method according to claim 1, wherein a cell at said state 1 is an endoderm derived cell and a cell at said state 2 is a cell of the thymus, thyroid, para-thyroid glands, larynx, trachea, lung, lining of the respiratory tract, urinary bladder, vagina, urethra, gastrointestinal organs, liver, pancreas, gut epithelium or the lining of the gastrointestinal tract.

12. A method according to claim 1, wherein a cell at said state 1 is a mesoderm derived cell and a cell at said state 2 is a cell of the smooth muscle, striated muscle, skeletal muscle, cardiac muscle, connective tissue, bone, cartilage, kidney, urogenital system, adrenal cortex, heart, blood vessels, bone marrow or lymphatic tissue or a haematopoietic cell.

13. A method according to claim 1, wherein a cell at said state 1 is a haematopoetic stem cell and a cell at said state 2 is a haematopoietic progenitor cell, hepatocyte, cholangiocyte, red blood cell or white blood cell.

14. A method according to claim 1, wherein a cell at said state 1 is a mesenchymal stem cell and a cell at said state 2 is a myocyte, adipocyte, chondrocyte, osteocyte, cardiomyocyte, neuron, bone marrow stromal cell or thymic stromal cell.

15. A method according to claim 1, wherein a cell at said state 1 is a neural stem cell or a human central nervous system stem cell and a cell at said state 2 is a muscle cell, neuron cell, astrocyte or oligodendrocyte.

16. A method according to claim 1, wherein a cell at said state 1 is isolated from a stromal vascular cell fraction of processed lipoaspirate and a cell at said state 2 is an adipocyte precursor, osteocyte precursor, chondrocyte precursor or myocyte precursor cell.

17. A method according to claim1, wherein a cell at said state 2 is an endocrine pancreatic cell.

18. A method according to claim 1, wherein a cell at said state 1 is a cell from the blastocyst inner cell mass and a cell at said state 2 is an endocrine pancreatic cell.

19. A method according to claim 1, wherein a cell at said state 1 is a nestin-positive islet-derived progenitor cell and a cell at said state 2 is an endocrine pancreatic cell or a hepatic cell.

20. A method according to claims 17 to 19, wherein said pancreatic cell produces insulin.

21. A method according to claim 20, wherein said pancreatic cell produces insulin in a glucose responsive manner.

22. A method according to claim 1, wherein a cell at one of said states is a chondrocyte.

23. A method according to claim 1, wherein cells at said state 1 are fully differentiated chondrocytes and cells at state 2 are dedifferentiated and/or expanded.

24. A method according to claim 22 to 23, wherein said chondrocytes are isolated from a human cartilage sample.

25. A method according to claim 1, wherein a cell at one of said states is a circulatory skeletal blood cell and said cell at the other state is an adipocyte or an osteocyte.

26. A method according to claim 1, wherein a cell at one of said states is an angioblast cell from the bone marrow and said cell at the other state is a cell of a newly formed blood vessel or a mature endothelial cell.

27. A method according to the preceding claims, wherein the DNA sample is taken from a source such as a cell culture or a tissue culture.

28. A method according to the preceding claims, wherein said conditions are characterized as allowing the cells and/or cell cultures to grow on scaffolds or otherwise in 3 dimensional conditions.

29. A method according to the preceding claims, wherein said conditions include the feeding of said cells on one or several reduced media or supplemented media.

30. A method according to the preceding claims, wherein said supplemented media contain growth or differentiation inducing factors, natural serums, natural extracts, synthetic supplements, recombinant growth factors or chemicals, which induce growth or differentiation, or a mixture of any of those.

31. A method according to the preceding claims, wherein said conditions include the feeding of cells on a feeder cell layer.

32. A method according to the preceding claims, wherein said conditions are **characterized by** a specified temperature, humidity, light, electrical field, magnetic field, O2, N2 and/or CO2 concentration.

33. A method according to the preceding claims, wherein said conditions include the treatment of said cells at state 1 with an effective amount of a reagent which is able to modify said cell's DNA methylation status.

34. A method according to the preceding claims, wherein said conditions include the treatment of said cells at state 1 with an agent involved in DNA methylation belonging to the group of 5-aza-2'-deoxycytidine, Trichostatin A, lankacidin, benzenamine, cyclohexane acetic acid, blue platinum uracil, methyl 13-hydroxy-15-oxo-kaurenoate, sulfonium, euphornin D, octadecylphosphoryl choline, gnidimarcin, and aspiculamycin HCl.

35. A method according to the preceding claims, wherein said conditions include the treatment of said cells at state 1 with an agent involved in DNA methylation belonging to the group consisting of inhibitors of DNA methylation and histone deacetylation, topoisomerase II, and DNA synthesis.

36. The use of the method according to claims 1-35 for improving the tissue engineering process.

37. The use of the method according to claims 1-35 for monitoring a cell differentiation process.

38. The use of the method according to claims 1-35 for monitoring the differentiation of cell lines derived from in vitro sources and cell lines derived from in vivo and/or autopsy sources.

39. The use of the method according to claims 1-35 for monitoring a process comprising several steps of transition of a cell from one state into another.

40. The use of the method according to claims 1-35 for validation of engineered tissue cells.

41. The use of the method according to claims 1-35 to distinguish between omnipotent cells and more differentiated cells.

42. The use of the method according to claims 1-35 for ensuring homogeneity of the cultured cells.

43. The use of the method according to claims 1-35 for detecting contamination of differentiated cells or engineered tissue with uncontrolled proliferating cells, such as progenitor or stem cells.

44. The use of the method according to claims 1-35 for identification of a tissue's cell of origin.

45. The use of the method according to claims 1-35 to ensure that the engineered tissue is derived from a specifically defined cell source.

46. The use of the method according to claims 1-35 for post surgery evaluation of the development of tissue transplanted into a patient.
